# EUROPÄISCHE PATENTSCHRIFT

(11) **EP 1 276 890 B1**
(45) Veröffentlichungstag und Bekanntmachung des Hinweises auf die Patenterteilung: **02.01.2008**
(21) Anmeldenummer: 01940346.8
(22) Anmeldetag: 19.04.2001
(51) Int. Cl.: C12P 7/62, C12P 7/56, C08G 63/08

(54) **VERFAHREN ZUR HERSTELLUNG VON POLYMILCHSÄURE UND VORRICHTUNG HIERZU**
METHOD FOR PRODUCING POLYLACTIC ACID AND CORRESPONDING DEVICE
PROCEDE PERMETTANT DE PRODUIRE DE L'ACIDE POLYLACTIQUE ET DISPOSITIF CORRESPONDANT

(30) Priorität: 20.04.2000 DE 10020898
(43) Veröffentlichungstag der Anmeldung: 22.01.2003
(73) Patentinhaber: Uhde Inventa-Fischer GmbH, 13509 Berlin (DE)
(72) Erfinder: GERKING, Lüder, 14195 Berlin (DE); HAGEN, Rainer, 13465 Berlin (DE); RICHTER, Klaus, 04209 Leipzig (DE); IDLER, Frank, 14471 Potsdam (DE); REIMANN, Winfried, 14473 Potsdam (DE); HANZSCH, Bernd, 14542 Geltow (DE)
(74) Vertreter: Pfenning, Meinig & Partner GbR
(86) Internationale Anmeldenummer: PCT/EP2001/004482
(87) Internationale Veröffentlichungsnummer: WO 2001/081610

(56) Entgegenhaltungen:
- DE-A1- 19 628 472
- DE-C1- 19 537 365
- US-A- 5 357 035
- JAVANAINEN P ET AL: "LACTIC ACID FERMENTATION ON BARLEY FLOUR WITHOUT ADDITIONAL NUTRIENTS" BIOTECHNOLOGY TECHNIQUES, Bd. 9, Nr. 8, 1995, Seiten 543-548, XP001012520 ISSN: 0951-208X
- HOFVENDAHL K ET AL: "Simultaneous enzymatic wheat starch saccharification and fermentation to lactic acid by Lactococcus lactis." APPLIED MICROBIOLOGY AND BIOTECHNOLOGY, Bd. 52, Nr. 2, August 1999 (1999-08), Seiten 163-169, XP002180432 ISSN: 0175-7598
- "Verfahren zur Produktion und Nutzung nachwachsender Rohstoffe" JAHRESBERICHTE DES ATB, [Online] Juni 2000 (2000-06), XP002180433 Gefunden im Internet: <URL:http://www.atb-potsdam.de/publikation en/Jahresberichte/jabe99/Teil_II_FSP_6.pdf > [gefunden am 2001-10-17]
- PAYOT T ET AL: "LACTIC ACID PRODUCTION BY BACILLUS COAGULANS-KINETIC STUDIES AND OPTIMIZATION OF CULTURE MEDIUM FOR BATCH AND CONTINUOUS FERMENTATIONS" ENZYME AND MICROBIAL TECHNOLOGY, STONEHAM, MA, US, Bd. 24, Nr. 3/4, 15. Februar 1999 (1999-02-15), Seiten 191-199, XP001024151 ISSN: 0141-0229 in der Anmeldung erwähnt

## Beschreibung

Die Erfindung betrifft ein Verfahren zur Herstellung von Polymilchsäure aus fermentativ hergestellter Milchsäure wobei als Rohstoff stärkehaltige landwirtschaftliche Prudukte, vorzugsweise Getreide, verwendet wird. Die Erfindung betrifft weiterhin eine Vorrichtung zur Durchführung dieses Verfahren.

Die Herstellung von Polymilchsäure (Polylactid) ist mehrfach beschrieben worden. Milchsäurebakterien benötigen für ihr Wachstum neben Vitaminen vor allem stickstoffhaltige Stoffe, wie Aminosäuren und Peptide. Als Quelle dieser Substanzen haben sich Hefeextrakt und Pepton bewährt. Die auf dieser Basis von J.C. De Man et al. (J. Appl. Bacteriol. 23 (1960), 130-135) vorgeschlagene MRS-Nährlösungsrezeptur wird inzwischen in allen Laboratorien der Welt für die Kultivierung von Milchsäurebakterien verwendet. Für die Versorgung von Zellkonzentrationen > 10 g/l muß der Anteil von Hefeextrakt und Pepton im Medium jedoch deutlich erhöht werden (Amrane,A. et al.: World J. Microbiol.& Biotechnol. 14(1998), x - y). Dadurch werden beide Einsatzstoffe zu Kostenfaktoren in technischen Anwendungen. In einer kontinuierlichen Produktionsanlage mit Zellrückhaltung kann so allein der Aufwand für Hefeextrakt bis zu 38 % der Betriebskosten ausmachen (Tejayadi, S. und Cheryan,M.: Appl. Microbiol. Biotechnol. 43(1995), 242-248). Eine Möglichkeit zur Kostensenkung wird darin gesehen, daß in einem Zweistufenprozeß zuerst eine wuchsstoffreiche und dann eine wuchsstoffarme Nährlösung verwendet wird (Olmos-Dichara, A. et al.: Biotechnol. Lett. 19(1997), 709-714). Die beste Alternative dazu ist aber die Substitution der teuren Hefeextrakt und Pepton-Preparationen durch billigere Wuchstoffquellen. In der Literatur werden dafür u.a. Molke-Proteinkonzentrat (Bury,D. et al.: Int. Dairy J. 8(1998), 149-151) und Autolysat von Brauereihefe (Selmer-Oisen,E. et al.: Milchwissenschaft 53(1998), 367-370) vorgeschlagen. Shamala, T.R. et al. (Enzyme Microb. Technol. 9(1987), 726-729) erzielten befriedigende Ergebnisse mit Weizenkleiehydrolysaten im niederen Produktivitätsbereich diskontinuierlicher Fermentationen. Der Einsatz nichthydrolysierter neutraler Weizenkleieextrakte brachte dagegen wesentlich schlechtere Ergebnisse. Auch Weizenmehlhydrolysate wurden auf ihre Eignung als Wuchstoffquelle untersucht (Hofvendahl,K. et al.: Enzyme Microbial Technol. 20(1997), 301-307). Dabei stellte sich aber heraus, daß zur Erzielung hoher Produktivitäten dennoch die Zufuhr von Hefeextrakt notwendig war.

In einem Herstellungsverfahren (PCT WO 98/28433) wird Molkeprotein in den Fermentor eingebracht und dort mit Hilfe von Proteasen hydrolysiert. Ein weiteres Verfahren (PCT WO 98/212611) verwendet Maisquellwasser und Getreide-Glutenfiltrat als Wuchsstoffquelle, wobei aber zur Neutralisation des darin enthaltenen SO₂-Anteils die Zugabe von Wasserstoffperoxid erforderlich ist.

In einer anderen Arbeit (Payot,T. et al.: Enzyme Microb. Technol. 24(1999), 191-199) wird ein Bakterienextrakt aus der Biomasse von Bacillus coagulans zur Teilsubstitution von Hefeextrakt verwendet. Zu diesem Zweck werden nach Beendigung einer Batch-Fermentation die gebildeten Bakterienzellen durch Zentrifugation abgetrennt, gewaschen und in einer Kugelmühle zerstört. Das erhaltene Homogenisat wird dann unter Zusatz von 6 n Schwefelsäure bei 90°C für 2 h hydrolysiert, anschließend mit 6 n Ammoniaklösung neutralisiert und durch Zentrifugation von Feststoffanteilen befreit. Die Lösung wird durch Sprühtrocknung zu einem Konzentrat aufgearbeitet.

Diese Verfahrensweise birgt den Nachteil in sich, daß neben der Fermentation ein separater Prozeß zur Herstellung des Bakterienextraktes erforderlich ist und daß bei der angegebenen Verfahrensweise größere Mengen an Sulfat- und Ammoniumionen über den Extrakt in das Fermentationsmedium gelangen, die bei der Aufarbeitung zu hochreiner Milchsäure aus dieser durch aufwendige Maßnahmen wieder entfernt werden müssen.

Die US 5 247 059 beschreibt ein Verfahren zur Herstellung von gereinigtem Lactid und Lactidpolymeren. Nach dem vorstehend erwähnten US-Patent wird von Milchsäure, die aus der Fermentation gewonnen worden ist, ausgegangen und diese Milchsäure wird mit einem Verdampfer auf eine 85 %ige Milchsäure hochkonzenriert. Anschließend wird ein Präpolymer gebildet und die erhaltene Polymilchsäure mit einem Molekulargewicht von 100 bis 5000 in einen Lactidreaktor zugeführt, wodurch als Rohprodukt Lactid erhalten wird. Dieses Lactid wird gereinigt (> 99 %) und dann einer Ringöffnungspolymerisation unterzogen.

Nachteilig bei diesem Prozeß ist, daß von einer lediglich 15 %igen Milchsäure ausgegangen wird und daß das mit diesem Verfahren erreichbare Molekulargewicht noch ungenügend ist.

Javanainen et al. beschreibt in Biotechnology Techniques 1995, 9, 543-548 die fermentative Herstellung von Milchsäure aus stärkehaltigem landwirtschaftlichem Gerstenmehl. Dabei wird Gerstenmehl mit Glucoamylase und α-Amylase enthaltenden Gerstenmehl hydrolysiert. Anschließend wird das hydrolysierte Gerstenmehl als Medium zur fermentativen Herstellung von Milchsäure mittels einer Mischkultur enthaltend Lac*tobazillus amylovorus* und *L. casei* benutzt. Bei dem Verfahren werden keine weiteren Zusatzstoffe wie Stickstoff, Vitamine oder Mineralien benötigt.

Hofvendahl et al. beschreibt in Micobiol. Biotechnol. 1999, 52, 163-169 die fermentative Herstellung von Milchsäure aus stärkehaltigem landwirtschaftlichem Weizenmehl. Dabei wird Weizenmehl mit α-Amylase und Amyloglucosidase hydrolysiert und mit einer Protease peptonisiert. Das behandelte Weizenmehl wir anschließend als Medium zur fermentativen Herstellung von Milchsäure mittels benutzt. Dabei enthält das Weizenmehl Proteine, Vitamine, Lipide, Mineralien und Stärke. Es sind keine weiteren Zutaten nötig. Vorteilhaft ist es, die Hydrolyse und die Fermentation separat durchzuführen.

Ein weiteres Verfahren zur Herstellung von Polymilchsäure ausgehend von einer Milchsäure enthaltenden Präparation beschreibt die Patentschrift US-A 5,357,035. Das Verfahren umfasst eine Hochreinigung und Konzentration der Milchsäure, Herstellung eines Präpolymers, cyclisierende Depolymerisation, Reinigung des Lactids und Ringöffnungspolymerisation des Lactides zur Herstellung von Polymilchsäure.

Weiterhin wird in der DE 195 37 365 C1 ein Verfahren zur Stabilisierung der Schmelzviskosität von Zinn-, Titan- und/oder Zirkoniumverbindungen als Katalysator und/oder Initiator enthaltender aliphatischer oder aromatisch-aliphatischer Homo- oder Copolyester, wobei der bei der Synthese oder bei der Wiederaufarbeitung der Polyester entstehenden Schmelze ein Maskierungsmittel, enthaltend eine Verbindung mit mindestens einem Tropolonring offenbart.

In einem weiteren Verfahren (DE 196 284 72) wird die Herstellung von aliphatischen Polestern und/oder Copolyestern mittels einer Polyreaktion von mindestens einem Monomer aus der Gruppe der Lactide, Lactone, cyclischen Carbonate und cyclischen Anhydride offenbart.

Ausgehend hiervon ist es Aufgabe der vorliegende Erfindung ein neues Verfahren und eine entsprechende Vorrichtung vorzuschlagen mit der kontinuierlich in hohen Ausbeuten Polylactid mit einem hohen Molekulargewicht hergestellt werden kann.

Die Aufgabe wird durch die kennzeichnenden Merkmale des Patentanspruches 1 und in Hinblick auf die Vorrichtung durch die kennzeichnenden Merkmale des Patentanspruches 20 gelöst.

Erfindungsgemäß wird somit das biologisch abbaubare Polylactid durch Polymerisation des zyklischen Dimers der Milchsäure gewonnen. Die Milchsäure entstammt einem Fermentationsprozeß, dessen Ausgangsstoff stärkehaltige landwirtschaftliche Produkte, insbesondere Getreide, sind, wobei diese einer Stärkehydrolyse unterzogen werden und der bei der Stärkehydrolyse anfallende Restfeststoff zusätzlich einer alkalischen Extraktion unterzegen und der erhaltene Protein extrakt als Wuchsstoffquelle dem Fermentor zugeführt wird. Somit besteht der Prozess aus einem bioverfahrenstechnischen Teil mit Hydrolyse, Fermentation und membrantechnischer Reinigung der Milchsäure und einem Polymerisationsprozessen vergleichbaren Abschnitt.

Ausgangsstoff ist Getreidemehl, insbesondere Roggenmehl, oder genauer die darin enthaltene Stärke. Die hydrolysierte Stärke wird enzymatisch zu Glucose abgebaut und zu wäßriger Milchsäure fermentiert. Die Milchsäure muß anschließend gereinigt und aufkonzentiert werden, bevor sie zu einem ersten Präpolymer polykondensiert werden kann. Dieses Präpolymer wird unter geeigneten Bedingungen zu dem eigentlichen Ausgangsstoff der Polymerisation, dem zyklischen Dimer der Milchsäure (Dilactid), depolymerisiert.

Unter dem Einfluß eines Katalysatores (z.B. Zinnoctoat) und mit Hilfe der als Startzentren wirkenden Hydroxylgruppen kann das Dilactid nun zum Polylactid polymerisiert werden. Die Qualität des Polymers hängt jedoch ganz entscheidend von der Reinheit des eingesetzten Dilactids ab, weshalb vor der Ringöffnungspolymerisation eine rektifikative Reinigung des Dilactids vonnöten ist.

Die anschließende Entmonomerisierung der Polylactidschmelze soll frühzeitige Abbauvorgänge im Endprodukt verhindern.

Wesentliches Element der Erfindung ist die Gewinnung von hochreiner Milchsäure durch eine kontinuierliche Hochleistungs-Fermentation. Nachfolgend wird dieser Teil des Verfahrens noch genauer beschrieben.

Zu Beginn des Prozesses steht die Vermengung von Getreidemehl, bevorzugt Roggenmehl und deionisiertem Wasser. Dies kann in einem einfachen Rührkessel geschehen. Wichtig ist das behutsame Verrühren und die Vermeidung hoher Wassertemperaturen, da ansonsten aufsteigender Wasserdampf mit dem Mehl verkleistern oder Klumpen bilden könnte. Nach bisheriger Praxis war es unerläßlich, bei kontinuierlichen Hochleitungsfermentationen externe Wuchsstoffquellen vorzugsweise Pepton und Hefeextrakt in erheblicher Menge zuzusetzen. Die dafür aufzuwendenden Kosten stellen die Wirtschaftlicheit solcher Verfahren in Frage. Außerdem werden mit Zusatz solcher Quellen störende Fremdstoff, z.B. Fremdionen (insbesondere Chloride) eingeschleppt, die die Reinigung des gebildeten Natriumlactats bzw. der Milchsäure durch Membrantrennprozesse sehr erschweren und außerdem bei ungenügender Reinigung die Ausbeute an Polylactid in einem nachfolgenden Polymerisationsprozess bis zur Unwirtschaftlichkeit herabsetzen. Entgegen der bisherigen Praxis wird nach dem erfindungsgemäßen Verfahren beim Verfahrensschritt a) keine externe Wuchsstoffquelle, wie Hefeextrakt oder Pepton verwendet, sondern die Wuchsstoffquelle wird aus dem Ausgangsstoff selbst, nämlich dem Getreide, gewonnen. Die Wuchsstoffe (Peptide, Aminosäure, Vitamine, Salze), die die Milchsäurebakterien bei der Fermentation neben einer verwertbaren Kohlenstoffquelle benötigen, stammen somit gemäß der Erfindung aus dem Ausgangsstoff.

Überraschenderweise wurde gefunden, daß alkalische Proteinextrakte aus Getreidemehl bzw. Getreideschrot bereits einen vollwertigen Ersatz für Pepton bzw. Hefeextrakt darstellen, wenn auf eine zusätzliche hydrolytische Spaltung durch Proteinasen verzichtet wird. Der Effekt kann noch dadurch verstärkt werden, daß die gebildete überschüssige Biomasse im Prozeß gezielt lysiert wird, wodurch bestimmte essentielle Wuchsstoffe wieder oder zusätzlich freigesetzt werden. Auf diese Weise kann sowohl auf Pepton als auch auf Hefeextrakt in Hochleistungsfermentationen völlig verzichtet werden.

Wenn Getreide als Rohstoff eingesetzt wird, kann die Proteinextraktion mit dem Stärkehydrolyseprozeß gekoppelt werden. Es besteht die Möglichkeit, eine Teilmenge des Mehls bzw. Schrotes für die Extraktgewinnung einzusetzen und die dabei zurückbleibende Stärkefraktion in die Verflüssigungsphase der Hydrolyse einzubringen. Eine Alternative dazu ist die alkalische Extraktion des bei der Stärkehydrolyse generell anfallenden Rest-Feststoffes, der vom Hydrolysat abgetrennt wird.

Die Lyse eines Teils der im Fermentationssystem befindlichen Biomasse muß möglichst vollständig und so erfolgen, daß dadurch keine Schädigung der für die Aufrechterhaltung des Fermentationsprozesses benötigten aktiven Zellfraktion eintritt. Hierzu wird ein Teil der Biomasse aus dem Fermentor in einen Kreislauf geführt, dort lysiert und das erhaltene Lysat wieder in den Fermentor zurückgeführt. Außerdem ist es möglich, die Überschußbiomasse abzutrennen und außerhalb des Fermentationssystems zu lysieren und den so erhaltenen Extrakt in das Fermentationssystem zurückzuführen.

Die auf diese Weise gewonnene Milchsäure wird dann wie bereits eingangs ausgeführt, einer Hochreinigung unterzogen und anschließend dann aufkonzentriert und der Polymerisation unterworfen.

Die Erfindung betrifft weiterhin eine entsprechende Vorrichtung zur Durchführung des Verfahrens.

Die Erfindung wird nachfolgend anhand von Figuren und Ausführungsbeispielen näher erläutert.

### Es zeigen:

- Fig. 1: das Schema der Proteinextrakterzeugung aus Getreidemehl/-schrot,
- Fig. 2: das Schema der Proteinextrakterzeugung aus Rückständen der Stärkehydrolyse,
- Fig. 3: Prozeßschema einer kontinuierlichen Fermentation mit Bakterienextrakterzeugung,
- Fig. 4a und b: den schematischen Verfahrensablauf einer kompletten Vorrichtung zur Erzeugung von Polylactid,
- Fig. 5: Wachstum von Lactobacillus rhamnosus 4759 in peptonlosem MRS-Medium mit Zugabe von alkalischem Roggenproteinextrakt (2) im Vergleich zum Wachstum in MRS-Medium (1) und MRS-Medium ohne Pepton (3),
- Fig. 6: Wachstum von Lactobacillus rhamnosus 4758 in hefeextraktlosen MRS-Medien mit Zusatz von Bakterienextrakten verschiedener Konzentrationen (erzeugt aus Zellsuspensionen mit Biotrockenmassegehalten von 15,7 g/l (3) bzw. 78,4 g/l (2) im Vergleich zum Wachstum in MRS-Medium (1) und MRS-Medium ohne Hefeextrakt (4),
- Fig. 7: Substrat- und Produktkonzentrationen einer Batch-Fermentation von Lactobacillus paracasei 160111 auf Roggenmehlhydrolysat bei Zugabe von Proteinextrakt aus Roggenmehl und Bakterienextrakt aus eigener Biomasse.

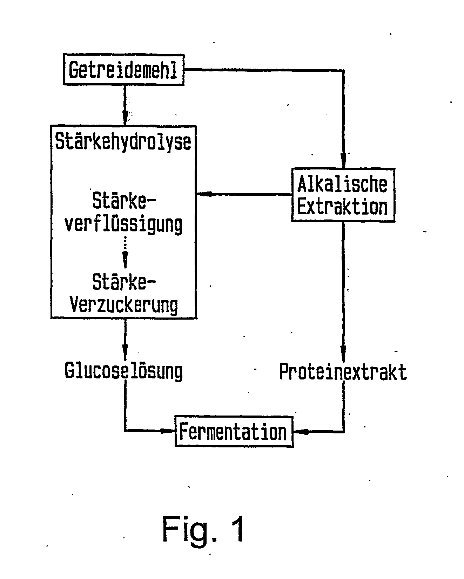

Figur 1 zeigt im Schema die Proteinextrakterzeugung aus Getreidemehl und Schrot. Danach wird eine Teilmenge des Mehls bzw. Schrots für die Extraktgewinnung direkt der alkalischen Extraktion zugeführt und dann der erhaltene Proteinextrakt in den Fermentor zur Fermentation überführt. Bevorzugt ist es dabei, daß ein Zehntel bis zur Hälfte, vorzugsweise ein Viertel des Getreidemehls der alkalischen Extraktion zugeführt wird und der Rest in der Stärkehydrolyse eingesetzt wird.

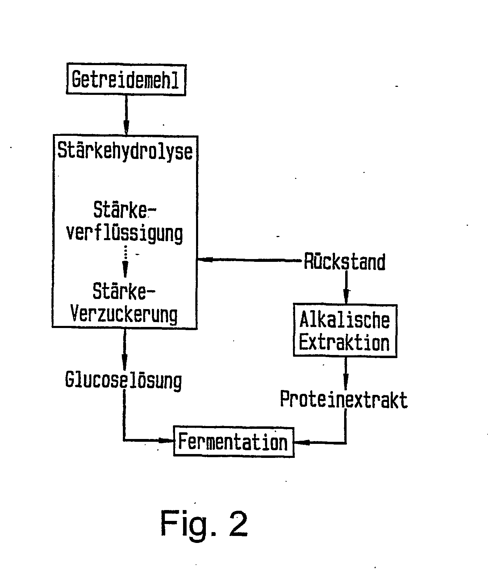

Eine Alternative hierzu' ist die alkalische Extraktion des bei der Stärkehydrolyse generell anfallenden Restfeststoffes der vom Hydrolysat abgetrennt wird (Fig. 2).

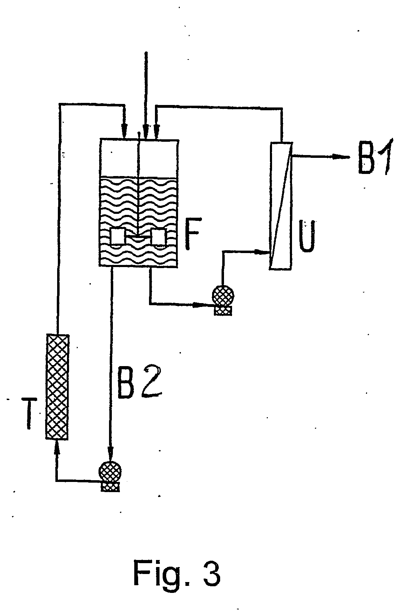

Figur 3 zeigt ein Prozeßschema einer kontinuierlichen Fermentation mit Bakterienextrakterzeugung. In einem Fermentationssystem, das aus einem Fermentor F und einem im Außenkreislauf befindlichen Ultrafiltrationsmodul U besteht, wird die Biomasse zurückgehalten und allmählich im System angereichert. Da für eine effektive Milchsäureproduktion eine optimale Konzentration der aktiven Biomasse eingehalten werden muß, ist man normalerweise gezwungen, neben dem Filtratausgang B1 noch einen zweiten Ausgang B2 vorzusehen. Letzterer dient dazu, die überschüssige Zellmmasse aus dem Fermentor zu entfernen. Dieser zweite Ausgangsstoffstrom wird erfindungsgemäß zur Zellextraktgewinnung genutzt, indem er über eine thermische Hydrolysestufe T geführt wird, wo die Zellen durch Thermolyse zerstört werden. Das Lysat wird dann nach Abkühlung in den Fermentor zurückgeführt. Außerdem besteht die Möglichkeit, diesem Stoffstrom vor der thermischen Behandlung lysierende Enzyme zuzusetzen oder die Lyse mit Hilfe von Strahlung, insbesondere Mikrowellenstrahlung, auszuführen.

Die erfindungsgemäße Verfahrensweise bietet gleichzeitig auch eine neue Möglichkeit zur Konstanthaltung der Konzentration der aktiven Biomasse im Reaktor auf optimalem Niveau, wenn durch entsprechende Regelungssysteme (z.B. durch einen geeigneten Trübungssensor) dafür gesorgt wird, daß in der Zeiteinheit gerade nur soviel Biomasse lysiert wird, wie im gleichen Zeitraum zugewachsen ist. In diesem Fall wird der Lysatstrom vor der Rückführung in den Fermentor von den verbliebenen Feststoffanteilen der Fermentation, z.B. durch Filtration, befreit.

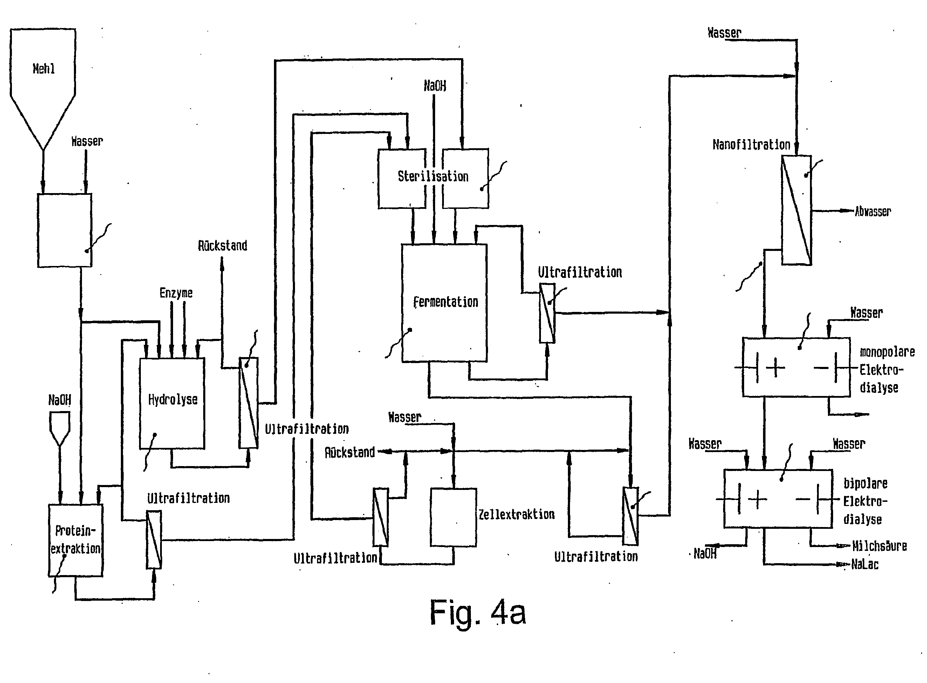

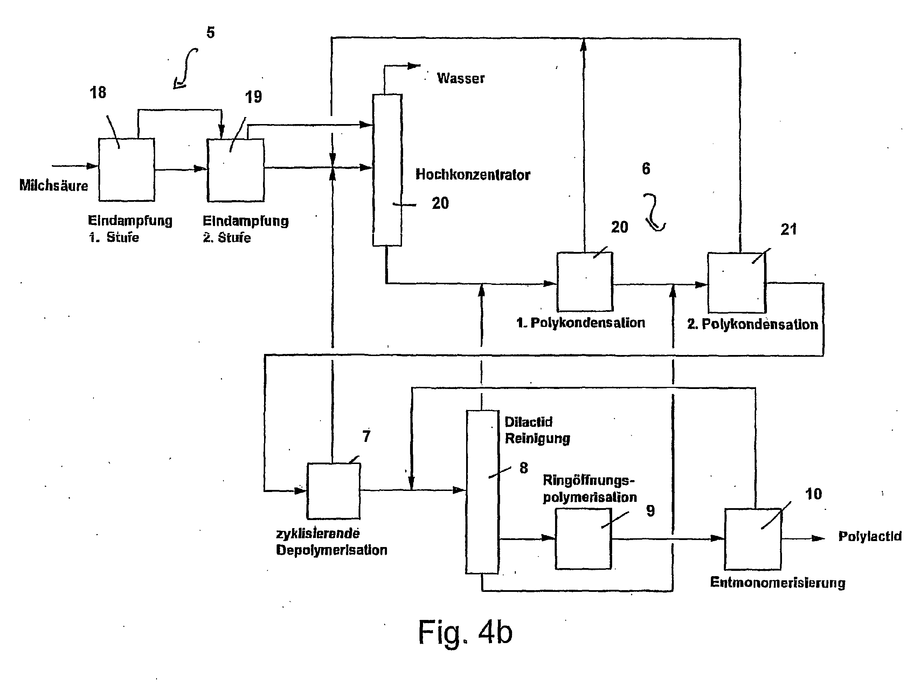

Die Figur 4 zeigt schematisch den Prozeßablauf der Herstellung.

Zu Beginn des Prozesses steht die Vermengung von Getreidemehl, bevorzugt Roggenmehl in einer Mischeinrichtung 1. Erfindungsgemäß wird somit ein Teil, bevorzugt etwa ein Viertel der so entstandenen Suspension der kontinuierlichen Proteinextraktion 11 zugeführt, etwa drei Viertel fließen direkt in die Hydrolyseeinrichtung 2. Diese kann diskontinuierlich oder kontinuierlich gestaltet sein. Im diskontinuierlichen Fall wird sie durch vor- und nachgechaltete Pufferbehälter quasi-kontinuierlich betrieben.

Die Hydrolyse ist ein zweistufiger enzymatischer Prozeß. In der ersten Stufe wird die Stärke unter Zugabe des Endoenzyms α-Amylase bei 80°C zwei Stunden lang verflüssigt. Anschließend erfolgt die Spaltung der hydrolysierten Stärke mit Hilfe des Exoenzyms Glucoamylase bei 55°C. Der zweite Schritt dauert etwa vier Stunden und wird in einer separaten Reaktionsstufe durchgeführt. Somit wird eine Totalverzuckerung der im Roggenmehl enthaltenen Stärke erreicht. Als Apparate können einfache, beheizbare Rührkessel eingesetzt werden.

Das Hydrolysat wird in einer Filterpresse von Feststoffen getrennt und der Fermentation zugeführt. Die abgetrennte Biomasse ist Rückstand und kann als Futtermittel oder eventuell zur Biogasproduktion verwendet werden.

Bei der Proteinextraktion im Extraktor 11 wird die Mehl-Wasser-Suspension unter Zugabe von Natronlauge während einer Dauer von acht Stunden auf einem' pH-Wert von 10 gehalten. Dadurch löst sich die Proteinfraktion und kann in einer anschließenden Ultrafiltration 30 als Permeat abgeschöpft und dem Fermentor zugesetzt werden. Der Filterkuchen wird mit Milchsäure neutralisiert und hydrolysiert. Druck und Temperatur entsprechen den Umgebungsbedingungen. Da das Gemisch gerührt werden muß, sollte der Behälter ein Rührkessel sein.

Weitere benötigte Wuchsstoffe wie u.a. Aminosäuren und Vitamine, können aus der bei der Fermentation 3 anfallenden, ultrafiltrierten Biomasse durch induzierte Lysis gewonnen werden. Dies wird mit der Ultrafiltrationseinrichtung 12 durchgeführt. Die induzierte Lysis kann auf verschiedene Arten geschehen, hier besteht sie aus einer 20-minütigen Erhitzung auf 95 C in einem beheizten Behälter. Eine Ultrafiltration trennt die Biomasse von dem Zellextrakt, der wieder in die Fermentation 3 fließt. Die anfallende Biomasse kann wiederum als Futtermittel genutzt werden.

Bevor der Nährstoff- und Proteinextrakt sowie das Hydrolysat in den Fermentor 3 gelangen, müssen sie kurzzeitig (20 min) bei 120°C (Mindesttemperatur 110°C) mit Sterilisatoren 13 sterilisiert werden. Hydrolysat und Wuchsstoffextrakte können nicht zusammen sterilisiert werden, um die bei diesen Temperaturen stattfindenden sog. Maillard-Reaktionen zwischen Stickstoffverbindungen (Proteine) und Glucose zu vermeiden, die insbesondere unerwünschte Verfärbung verursachen. Die Behälter 13 müssen für die Verweilzeit und Temperatur für die Sterilisation ausgelegt sein.

Die Fermentation stellt die Prozeßstufe dar, in der die Biokonversion von Glucose in Milchsäure erfolgt. Diese geschieht in den Milchsäurebakterienzellen nach einem komplizierten Ablauf von vielen metabolischen Einschrittreaktionen (Glycolyse), deren jede durch ein bestimmtes Enzym katalysiert wird. Es handelt sich um einen anaeroben Prozeß, bei dem die Bakterien Milchsäure bilden, um die für'ihre Vermehrung notwendige Energie zu gewinnen. Die mikrobielle Reaktion liefert also neben der Milchsäure auch Zellmasse und in ganz geringen Mengen andere Produkte.

Die Aktivität der Bakterienzellen hängt von einer Reihe von Prozeßparametern ab (Temperatur, pH-Wert, Osmolalität, Substratkonzentration, Wuchsstoffangebot usw.). Für den gewählten Stamm der Lactobacillus paracasei beträgt die optimale Temperatur 33°C und der pH-Wert 6,0. Zur Regulierung des pH-Wertes ist die Zugabe von Natronlauge erforderlich. Unter diesen Bedingungen entsteht die Milchsäure in Form von Natriumlactat. Die Verweilzeit beträgt typischerweise zwischen anderthalb und vier Stunden (Durchflußrate: 0,25 - 0,67). Beim Fermentor 3 muß darauf geachtet werden, daß er im Inneren eine möglichst glatte, von Ecken, Kanten und Rillen freie Oberfläche besitzt, damit die regelmäßig notwendige Sterilisation des Fermentors einfach und wirkungsvoll gehalten werden kann (Wasserdampfreinigung). Ein Rührwerk ist erforderlich.

Der Fermentorinhalt wird kontinuierlich ultrafiltriert, wobei Natriumlactat als Permeat anfällt. Das zellhaltige Retentat fließt wieder in den Fermentor 3. Um eine Aufkonzentrierung der Zellmasse zu verhindern, verlässt ein zweiter Produktstrom den Fermentor 3. Das darin enthaltene Natriumlactat wird in einer weiteren Ultrafiltrationsstufe 31 zurückgewonnen, das zellhaltige Retentat fließt nicht in den Fermentor, sondern in die bereits oben beschriebene Nährstoffextraktion.

Wesentlich beim erfindungsgemäßen Verfahren (Merkmal b) ist die Hochreinigung der Milchsäure. Erforderlich ist dies, da der ultrafiltrierte Fermentorablauf neben der Milchsäure in Form von Natriumlactat weitere Komponenten enthält, die insbesondere die spätere Polymerisation empfindlich stören und so sogar den ganzen Prozeß zum Erliegen bringen können. Als ungewünschte Inhaltsstoffe sind vor allem anorganische und organische Säuren (Essigsäure, Propionsäure, Schwefelsäure, Salzsäure) und deren Salze, Mono-, Di- und Oligosaccharide und Farbstoffe zu nennen.

Mit Hilfe der Nanofiltration 15 sollen in erster Linie die Chlorid-Ionen vom Natriumlactat getrennt werden. Chlorid-Ionen werden hauptsächlich mit den bei der Hydrolyse eingesetzten Enzymen in den Prozeß gebracht. Sie sind nicht nur bei den Polymerisationsreaktionen sehr störend, sondern greifen auch die Behältermaterialien stark an. Mit der verwendeten Membran können bis zu 98% der Chlorid-Ionen zurückgehalten werden.

Daneben permeieren weitere Ionen wie Sulfate und Phosphate durch die Membran, das Natriumlactat bleibt im Retentat.

Die monopolare Elektrodialyse 16 ist genauso wie die bipolare ein diskontinuierlicher Prozeß. Um trotzdem eine kontinuierliche Milchsäureproduktion zu ermöglichen, werden zwei Dialyseanlagen alternierend betrieben.

Die monopolare Elektrodialyse wird aus zwei Gründen eingesetzt:
1. Trennung der nichtionischen von den ionischen Komponenten und
2. Aufkonzentrierung des Natriumlactats.

Zu den nichtionischen Komponenten, die vom Natriumlactat getrennt werden, gehören insbesondere Stickstoff- und Phosphorverbindungen. Diese Stoffe befinden sich in der großen, anfallenden Menge Abwasser und verhindern eine vollständige Rückführung dieser Wassermenge in die Hydrolyse und Fermentation. Temperatur und Druck sind wie bei Umgebungsbedingungen.

Um schließlich Milchsäure aus dem Natriumlactat zu gewinnen, wird eine Elektrodialyse 17 mit bipolaren Membranen eingesetzt. Die Anlage besitz drei Kreisläufe:
➢ einen Salzkreislauf, in den das Natriumlactat eintritt,
➢ einen Laugenkreislauf, in den die Natrium-Ionen diffundieren und sich mit Hydroxid-Ionen zu Natronlauge verbinden
➢ und einen Säurekreislauf, in dem sich Lactat-Ionen mit Protonen zu Milchsäure verbinden.

Um den Strombedarf der Elektrodialyse 17 in wirtschaftlichen Grenzen zu halten, wird sie so gefahren, daß nicht das gesamte Natriumlactat zu Milchsäure umgewandelt wird. Diese Restmenge muß aus dem Prozeß ausgeschleust werden. Die erzeugte Natronlauge fließt zurück in die Fermentation, um dort den pH-Wert zu regeln.

Die Temperatur der zirkulierenden Lösungen sollte konstant auf 33 C gehalten werden. Die Zwischenlagerbehälter müssen somit gekühlt werden.

Die aus der bipolaren Elektrodialyse 17 kommende Milchsäure muß weiter aufkonzentriert werden (Merkmal c) Erfindungsgemäß wird eine zweistufige Eindampfung mit dem Eindampfer 18, 19 der Milchsäure vorgenommen. Hierbei wird die wäßrige Milchsäure zuerst bei Überdruck zum Sieden gebracht, wodurch ein Großteil des Wassers bereits verdampft. Anschließend kann dieser Wasserdampf als Heizmedium genutzt werden, um weiteres Wasser aus der verbleibenden Milchsäure bei niedrigerem Druck zu treiben. Die danach noch verbleibende Flüssigkeit wird in einem Roberts-Verdainpfer 20 mit aufgesetzter dreibödigen Rektifikationskolonne zu 90%iger, bevorzugt 95%iger Milchsäure aufkonzentriert (Hochkonzentration).

Die konzentrierte Milchsäure wird nun in zwei Reaktoren 20, 21 mit außenliegendem Umlaufverdampfer und unter Zusatz eines Katalysators zu einem Präpolymer polykondensiert. Die Vorkondensation findet bei zwei unterschiedlichen Drücken statt. Im ersten Reaktor 20 herrscht Umgebungsdruck oder sogar leichter Überdruck, um ein Verdampfen der Milchsäure zu verhindern. Ist der Großteil der Milchsäure zu einem höhersiedenden Oligomer polykondensiert, kann die Reaktion im zweiten Reaktor 21 unter Vakuum (50 mbar) weitergeführt werden. Das angelegte Vakuum erleichtert das Verdampfen des bei der Reaktion entstehenden Wassers aus der Schmelze und verhindert somit, daß die Reaktion durch Erreichen des chemischen Gleichgewichts zum Erliegen kommt. Die Verweilzeit in den Reaktoren beträgt drei und vier Stunden, die Temperaturen betragen 180 C und 190 C, jeweils im ersten und zweiten Reaktor. Das Präpolymer hat ein Molekulargewicht von 3400 g/Mol (2500 - 4000 g/Mol).

Die Depolymerisation (Merkmal d)) zum eigentlichen Monomer des Polylactids, dem Dilactid findet bevorzugt in einem Fallfilmverdampfer 7 statt. Das Präpolymer wird auf mehrere beheizte senkrechte Rohre verteilt und rieselt darin in einem dünnen Film herab. Die Temperatur wird auf ca. 210°C erhöht und der Unterdruck aus der Vorkondensation beibehalten (50 mbar). Die erhöhte Temperatur beschleunigt die Dilactidbildung, das Vakuum und der dünne Rieselfilm (< 1 mm) sorgen für ein schnelles Verdampfen des entstandenen Dilactids. Der Fallfilmverdampfer 7 wird als Umlaufverdampfer betrieben, um vollständige Benetzung der beheizten Fläche sicherzustellen.

Der dampfförmige Produktstrom des Fallfilmverdampfers 7 wird sofort teilkondensiert. Temperatur und Druck der Teilkondensation werden dabei so gewählt, daß das sich im Dampf befindende Wasser und ein möglichst großer Teil der Milchsäure dampfförmig bleiben. Das Dilactid wird fast vollständig kondensiert. Das Kondensat enthält nur noch geringe Menge an Milchsäure und Oligomeren, wie z.B. Lactoylmilchsäure, dem linearen Dimer der Milchsäure. Zusammen ergibt sich daraus die wichtige Hydroxylgruppenkonzentration, die typischerweise 57 meq beträgt.

Bei der Ringöffnungspolymerisation hängt das erreichbare Molekulargewicht und somit wichtige mechanische Eigenschaften des Polylactids von der Dilactidreinheit ab. Durch die restliche Milchsäure und Lactoylmilchsäure befinden sich Hydroxylgruppen im Dilactid. Diese stellen Startzentren der Polymerisation dar. Je höher die Konzentration an Hydroxylgruppen im Dilactid ist, desto mehr Polymermoleküle entstehen und desto geringer ist das erzielbare Molekulargewicht. Die angestrebte Hydroxylgruppenkonzentration beträgt 20 meq, was zu einem theoretischen Molekulargewicht von 50000 g/Mol führt. Die maximal erreichbare Dilactidreinheit mit dieser Kolonne beträgt 10 meq.

Die Konzentration der Hydroxylgruppen im Dilactid ist nach der zyklisierenden Depolymerisation noch zu groß. In einer Rektifikationskolonne 8 mit Seitenentnahme wird das kondensierte Dilactid auf die gewünschte Hydroxylgruppenkonzentration gereinigt. Gleichzeitig kann die Kolonne zur Kontrolle des Molekulargewichts genutzt werden.

Das verunreinigte Dilactid tritt in den oberen Teil der Kolonne ein und verlässt sie gereinigt im unteren Teil als dampfförmige Seitenentnahme. Der Dilactiddampf wird kondensiert bevor er in die Ringöffnungspolymerisationsreaktoren tritt. Kopfprodukt (148°C, 30 mbar) ist die restliche Milchsäure, über den Sumpf (172°C, 60 mbar) werden die höher siedenden Oligomere der Milchsäure und eventuell vorhandene sonstige Verunreinigungen entnommen. Die Kolonne wird aus thermodynamischen-Gesichtspunkten unter Vakuum (30 - 60 mbar) betrieben (Vermeidung zu hoher Temperaturen Verbesserung der relativen Flüchtigkeiten).

Die Ringöffnungspolymerisation (Merkmal f)) findet in einer Rührkesselkaskade 9 von zwei Reaktoren unter Zusatz eines Katalysators statt. Die Konzentration des Katalysators wird dabei vergleichsweise gering gehalten (5*10-5 mol Katalysator/mol Dilactid, Konzentrationsbereich: 2*10-4 bis 2*10-5 mol/mol), um hohe Molmassen zu erhalten und Nebenreaktionen zurückzudrängen. Die Reaktoren stehen unter Atmosphärendruck.

Bei der Ringöffnungspolymerisation handelt es sich um eine exotherme Reaktion. Um Temperaturen von über 240°C zu vermeiden (thermischer Abbau, Nebenreaktionen), muß ein Teil der entstehenden Reaktionswärme abgeführt werden. Im ersten Reaktor geschieht dies durch Eispeisen von unterkühltem Dilactid: Das Dilactid wird dabei soweit unterkühlt, daß sich im ersten Reaktor eine Temperatur von 200°C einstellt. Bei einer Verweilzeit von 2,5 Stunden polymerisieren dabei ca. 70 Prozent des Dilactids.

Der zweite Reaktor wird adiabat gefahren und die Verweilzeit so gewählt (2 h), daß der Dilactidumsatz schließlich 90 Prozent beträgt. Die Schmelzetemperatur steigt dabei auf 215 C.

Das Polylactid hat nun das angestrebte Molekulargewicht von ca. 50000 g/Mol.. Jedoch befinden sich noch ca. neun Prozent Monomer in der Schmelze. Ein über längere Zeit stabiles Polylactid soll aber nicht mehr als ein Prozent Dilactid enthalten. Es ist also eine Entmonomerisierung vorzunehmen. Diese wird wiederum durch die Tatsache erschwert, daß es sich bei der Ringöffnungspolymerisation um eine Gleichgewichtsreaktion handelt. Bei Temperaturen um 200°C liegt die Gleichgewichtskonzentration von Dilactid bei ca. fünf Prozent. Die Entmonomerisierung muß somit entweder sehr schnell erfolgen, um die Rückbildung von Dilactid zu minimieren, was allerdings bei den hohen Viskositäten sehr schwierig zu bewerkstelligen ist. Eine zweite Möglichkeit, die hier Verwendung findet, besteht darin, durch Zugabe eines Stabilisators (z.B. α-Tropolon, siehe Patentschrift DE 195 37 365 C1) den Katalysator zu blockieren und die Reaktion fast zum Erliegen zu bringen und so die schädliche Rückbildung von Dilactid zu vermeiden.

Im vorliegenden Prozeß findet die Entmonomerisierung (Merkmal g)) nach Zugabe des Stabilisators in zwei separaten Apparaten 10 statt. Im ersten Apparat wird die Schmelze auf einen Druck von 10 mbar entspannt, wobei der größere Teil des Monomers verdampft. Der Dilactidgehalt kann somit auf ungefähr 2% gesenkt werden. Allerdings fällt dabei auch die Temperatur auf 195 C, was zu einer Viskositätserhöhung auf rund 700 Pa*s führt. Um schließlich noch die letzten zwei Prozent Dilactid aus der hochviskosen Schmelze zu verdampfen, wird der Druck im zweiten Apparat, dem sog. Finisher, auf 2,5 mbar gesenkt.

Bevor die Schmelze weiter entmonomerisiert wird, erfährt sie eine Temperaturerhöhung, um zu hohe Viskositäten am Austritt des Finishers zu verhindern. Der Finisher besteht aus einer zylindrischen Reaktorhülle, die zu 20 - 30% ihres Volumens mit Polymerschmelze gefüllt ist. Um die Zylinderachse rotiert ein korbartiger Träger, auf dem senkrecht stehende ringförmige Scheiben angebracht sind. Die Scheiben tauchen mit einem Teil ihrer Fläche in die Schmelze ein.

Durch die Rotation wird die hochviskose Schmelze von den Scheiben mitgezogen und in Form eines Films dem Vakuum ausgesetzt. Das Prinzip eines geeigneten Finishers ist z.B. in der US 5 77 99 86 beschrieben.

Anstelle eines derartigen "liegenden" Finishers eignet sich auch ein sogenannter Dünnschichtverdampfer. Hier fließt die zu entmonomerisierende Schmelze an der Innenwand eines senkrecht stehenden, von außen beheizten Rohres herab. In der Rohrachse rotiert eine angetriebene Welle, die Wischelemente trägt, welche die Schmelze über die beheizte Fläche während des Herabfließens zu einem dünnen Film verstreichen. Die Bildung dünner Schichten und deren ständige Erneuerung erleichtern das Verdampfen des Monomers.

Mit den beispielhaft angegebenen Vorrichtungen wird eine sich ständig erneuernde, sehr große Oberfläche erhalten, welche für eine Entmonomerisierung auf 0,5 Prozent Monomergehalt notwendig ist. Die Temperatur der Schmelze sinkt dabei auf 190°C und die Viskosität steigt auf ca. 1440 Pa* s.

### Beispiel 1

In einem 10-1 Rührfermentor werden 1 kg Roggenmehl in 5 1 Wasser eingerührt bei gleichzeitiger Einstellung des pH-Wertes auf 10,0 ( Zugabe von 3N NaOH-Lösung ). Nach einer Rührdauer von 2 Stunden bei 20°C erfolgt die Abtrennung der stärkehaltigen Feststofffraktion durch Filtration. Der wäßrige Überstand wird als alkalischer Proteinextrakt dem Fermentationsmedium zugesetzt.

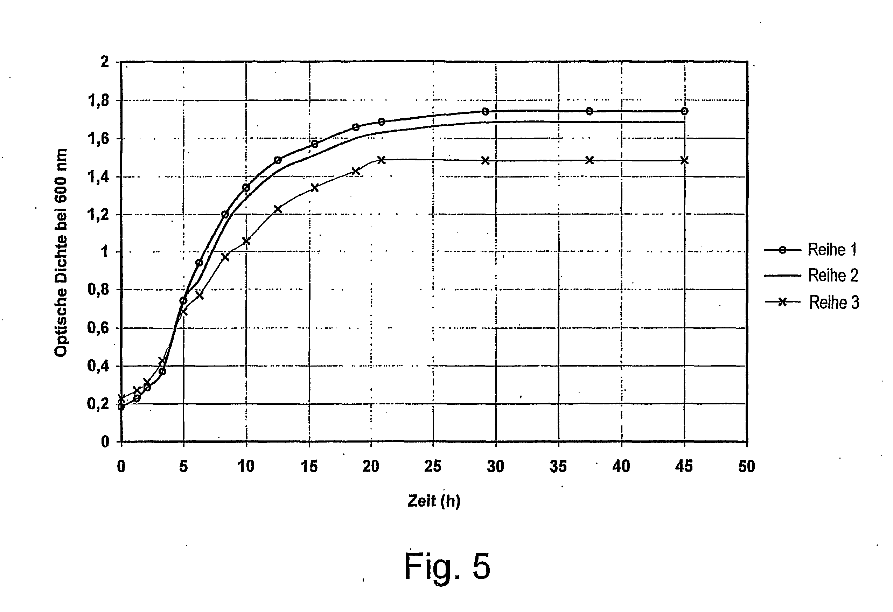

Die Wirksamkeit dieses Proteinextraktes läßt sich veranschaulichen am Wachstumsverhalten des Stammes Lactobacillus rhamnosus 4759 in Mikrotiterplatten des Gerätesystems Bioscreen (Firma Labsystems, Finnland). - Zu diesem Zweck wurden 0,24 ml eines Glucose - MRS-Mediums, das kein Pepton enthielt, mit 0,1 ml des hergestellten Proteinextraktes versetzt und mit 0,01 ml einer 18-h-Kultur des vorgenannten Stammes beimpft und unter Standardbedingungen (T=33°C, pH=6,0) kultiviert. Als Kontrollansätze kamen ein Voll-MRS-Medium und ein MRS-Medium ohne Pepton zum Einsatz. Die Wachstumskurven von Lactobacillus rhamnosus 4759 auf den drei beschriebenen Medien zeigt die Fig. 5. Die Wachstumskurven (1) und (2) unterscheiden sich nur geringfügig, d.h., daß in diesem Falle der Proteinextrakt als vollwertiger Ersatz des Peptons betrachtet werden kann.

### Beispiel 2

Zellmasse von Lactobacillus rhamnosus wird aus fermentierter Fermentorflüssigkeit durch Zentrifugation abgetrennt und in Wasser so resuspendiert, daß Zellsuspensionen mit Biomassetrockengehalten von 15,7 g/l und 78,4 g/l entstehen. Diese erhitzt man auf 60°C für 20 min, um die Zellen zu lysieren . Nach dem Abkühlen auf etwa 30°C und der Abtrennung der Zellwandreste wird die klare Lösung als Bakterienextrakt dem Fermentationsmedium zugesetzt.

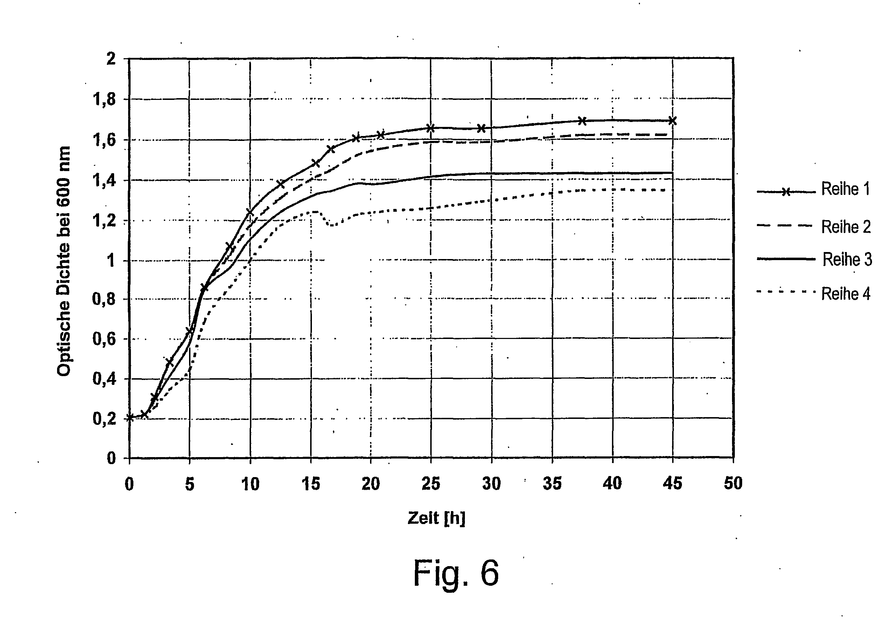

Die Wirksamkeit dieses Bakterienextraktes läßt sich veranschaulichen am Wachstumsverhalten des Stammes Lactobacillus rhamnosus 4759 in Mikrotiterplatten des Gerätesystems Bioscreen (Firma Labsystems, Finnland). Zu je 0,24 ml eines MRS-Mediums ohne Hefeextrakt wurden jeweils 0,1 ml eines der beiden Bakterienextrakte und 0,01 ml einer 18-h-Vorkultur des vorgenannten Stammes zugesetzt und bei T=33°C und pH=6,0 inkubiert. Als Kontrollansätze dienten ein Voll-MRS-Medium und ein MRS - Medium ohne Hefeextrakt. Die Wachstumskurven von Lactobacillus rhamnosus 4759 auf den vier genannten Medien zeigt die Fig. 6. Die Wachstumskurven (1) und (2) unterscheiden sich nur geringfügig, d.h., daß in diesem Falle der Bakterienextrakt als vollwertiger Ersatz des Hefeextraktes betrachtet werden kann.

### Beispiel 3

In einem 50-l Rührfermentor wurden 29 1 enzymatisch gewonnenes Roggenstärkehydrolysat (Glucosegehalt: 120 g/l) intern sterilisiert. Danach erfolgte unter aseptischen Bedingungen die Zugabe von 10 l des nach Beispiel 1 hergestellten Proteinextraktes, 6 l des nach Beispiel 2 erzeugten Bakterienzellextraktes sowie 1 l einer wäßrigen Lösung, die 96 g Dikaliumhydrogenphosphat, 4,8 g Magnesiumsulfat und 2,4 g Mangansulfat enthielt. Als Inoculum dienten 2 l einer Vorkultur des Stammes Lactobacillus paracasei 160111. Während des sich anschließenden Fermentationsprozesses wurden die Temperatur ständig bei 33 C und der pH-Wert bei 6,0 gehalten. Als Korrekturmittel für die pH-Regelung diente 30%ige Natronlauge..

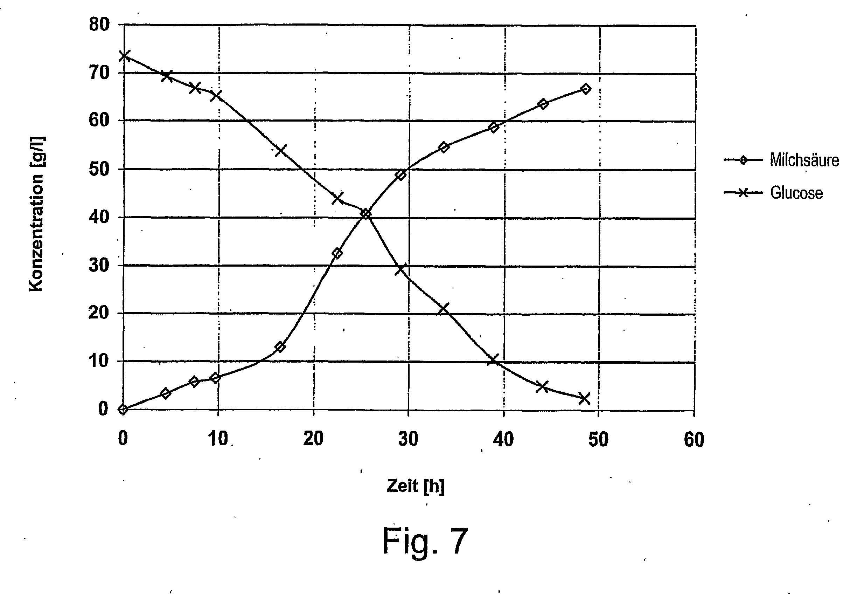

Nach 48 Stunden war die eingesetzte Glucose vollständig verbraucht und in Milchsäure umgewandelt (Fig. 7).

### Beispiel 4

. In einen 5-l Fermentor, der mit einem Ultrafiltrationsmodul im Außenkreislauf gekoppelt war, wurde mit einer konstanten Geschwindigkeit ein Salz-Nährmedium dosiert, das u.a. 50g/l Glucose, 10g/l Hefeextrakt und 10 g/l Pepton enthielt. Die gleiche Flüssigkeitsmenge verließ das Fermentationssystem zeitgleich als zellfreie Natriumlactatlösung über den Ausgang der Ultrafiltrationseinheit. Die mit dem Stamm Lactobacillus paracasei 160111 bei unterschiedlichen Durchflußraten ermittelten Produktivitäten der Milchsäureproduktion sind in der Tabelle 1 den Produktivitäten gegenübergestellt, die unter sonst gleichen Bedingungen mit einer Nährlösung erhalten wurden, die zu 10% aus Proteinextrakt nach Beispiel 1 bestand und 50g/l Glucose sowie 10g/l Hefeextrakt enthielt.

### Beispiel 5

In einen 50-l Fermentor, der mit einem Ultrafiltrationsmodul im Außenkreislauf gekoppelt war, wurde mit einer Geschwindigkeit von 6 1/h ein Salz-Nährmedium dosiert, das u.a. 40g/l Glucose, 10g/l Hefeextrakt und 10 g/l Pepton enthielt. Das Fermentationsmedium zirkulierte ständig zwischen Fermentor und Ultrafiltrationsmodul, wo die gleiche Flüssigkeitsmenge zeitgleich das Fermentationssystern als zellfreie Natriumlactatlösung verließ. Die mit dem Stamm Lactobacillus paracasei 160111 bei der Durchflußrate von D = 0,12 h⁻¹ erzielte Milchsäureproduktivität betrug 4,0 g/lh.

### Beispiel 6

In einen 50-l Fermentor, der mit einem Ultrafiltrationsmodul im Außenkreislauf gekoppelt war, wurde mit einer Geschwindigkeit von 6 1/h ein Roggenhydrolysat-Salz-Nährmedium dosiert, das zu 20% aus Roggenproteinextrakt gemäß Beispiel 1 bestand und 40 g/l Glucose enthielt. Das Fermentationsmedium zirkulierte ständig zwischen Fermentor und Ultrafiltrationsmodul, wo die gleiche Flüssigkeitsmenge zeitgleich das Fermentationssystern als zellfreie Natriumlactatlösung verließ. Gegenüber dem Beispiel 4 wurde das Fermentationsregime so verändert, daß die im Verlauf des Prozesses über die gewünschte Konzentration von 20 g/l hinaus zugewachsene Biomasse in einem zweiten Kreislauf für 5 Minuten auf 80°C erhitzt und dann als Lysat wieder in das Reaktorsystem zurückgeführt wurde. Im vorliegendem Falle betrug der Biomassezuwachs im Fermentor 2 g/lh , so daß stündlich aus dem Fermentorsystem jeweils 4,5 Liter, die etwa 100 g Biomasse enthielten, entnommen und auf die beschriebene Weise lysiert wurden. Die mit dem Stamm Lactobacillus paracasei 160111 bei der Durchflußrate von D = 0,12 h⁻¹ erzielte Milchsäureproduktivität betrug 3,95 g/1h.

### Beispiel 7

In einen 50-l Fermentor, der mit einem Ultrafiltrationsmodul im Außenkreislauf gekoppelt war, wurde mit einer Geschwindigkeit von 12,5 l/h ein Roggenhydrolysat-Nährmedium dosiert, das zu 20% aus Roggenproteinextrakt bestand und 50 g/l Glucose enthielt. In diesem Fall kam ein Roggenproteinextrakt zum Einsatz, dessen Wirkstoffanteil doppelt so hoch war wie der des nach Beispiel 1 erzeugten Extraktes. Als Ausgangsmaterial diente in diesem Fall nicht Mehl sondern der stärkefreie Rückstand aus der Stärkemehlhydrolyse, wobei das Feststoff-Wasser-Verhältnis bei der Extraktion zweimal so hoch war wie im Beispiel 1. Das Fermentationsmedium zirkulierte.ständig zwischen Fermentor und Ultrafiltrationsmodul, wo das der Dosiermenge entsprechende Flüssigkeitsvolumen zeitgleich das Fermentationssystern als zellfreie Natriumlactatlösung verließ. Die Biomassekonzentration im Reaktorsystem blieb dadurch konstant bei etwa 30 g/l, daß ständig ein Teilstrom der Fermentationsflüssigkeit, der über eine Flow-Injection-Trübungsmessung gesteuert wurde, über die thermische Lysestrecke (T=100 °C) geführt und nach Abtrennung der Zellrückstände mit Hilfe einer in den Prozeß integrierten Filtrationseinrichtung wieder in den Fermentor zurückgeleitet wurde.

Die mit dem Stamm Lactobacillus paracasei 160111 bei der Durchflußrate von D= 0,25 h⁻¹ erzielte Milchsäureproduktivität betrug 13,5 g/lh.

### Beispiel 8

Die zellfreie Natrium-Lactat-Lösung aus Beispiel 7 muss nun von Fremdstoffen gereinigt und in Milchsäure umgewandelt werden. Dabei wurde schon bei den Eingangsstoffen darauf geachtet, dass die Belastung an Fremdstoffen minimal ist. So lässt sich die Konzentration an Sulfat-Ionen im Fermentationsmedium durch den Einsatz von Magnesiumoxid anstatt Magnesiumsulfat und Milchsäure an Stelle von Schwefelsäure von 286 g/l auf 86 g/l senken. Der Eintrag der Chlorid-Ionen in den Prozess geschieht hauptsächlich durch das Enzym Termamyl 120 L. Hier kann der Chlorid-Gehalt mit Hilfe eines Ionentauschers von ursprünglich 675 g/l auf 212 g/l gesenkt werden.

Der Großteil der verbleibenden Chlorid-Ionen wird in der der Ultrafiltration folgenden Nanofiltration von der Natrium-Lactat-Lösung getrennt. Die Nanofiltration wird als dreistufige Diafiltration betrieben. Die Chlorid-Ionen diffundieren durch die Membran, die Lactat-Ionen verbleiben im Retentat. Auf diese Weise wird die Chlorid-Konzentration von 0,892 g/l im Kulturfiltrat auf 0,003 g/l im Konzentrat der dritten Nanofiltrationsstufe gesenkt.

Die anschließende monopolare Elektrodialyse dient der Aufkonzentrierung der Natrium-Lactat-Lösung sowie der Abscheidung der nichtionischen Komponenten. Insbesondere bleibt der Großteil des Restzuckers (reduzierende und nichtreduzierende Mono-, Di- und Ollgosaccharide) im Diluat. Die Gesamtphosphorkonzentration kann von 0,58 g/l im Zulauf auf 0,12 g/l im Konzentrat gesenkt werden. Für Stickstoff ergibt sich eine Reduktion von 1,04 g/l auf 0,131 g/l.

Wird der Restzucker nicht vollständig von der Milchsäure getrennt, sinkt die Ausbeute bei der Polymerisation erheblich: die verbleibenden Saccharide führen bei der zyklisierenden Depolymerisation zu einer starken Verkohlung des Präpolymers. Je nach Verunreinigung können dadurch bis zu 50 Prozent des eingesetzten Präpolymers verkohlen und damit unbrauchbar werden.

Schließlich wandelt die bipolare Elektrodialyse das Natrium-Lactat in wässrige Milchsäure und Natronlauge um. Letztere wird zurückgeführt und in der Fermentation zur pH-Regelung eingesetzt. Die erzeugte Milchsäure hat eine Konzentration von ca. 150 g/l. Die Konzentration an Chlorid-Ionen ist kleiner als 30 mg/l und die der Sulfat-Ionen kleiner als 10 mg/l. Die Konzentration des gesamten Stickstoffs sowie des Phosphors liegt jeweils unter 100 mg/l. Die Milchsäure besteht zu 95% aus L(+)- und zu 5% aus D(-)-Milchsäure.

### Beispiel 9

Die wässrige Milchsäure aus Beispiel 8 wird in einer zweistufigen Eindampfung mit anschließender Rektifikation aufkonzentriert. In der ersten Stufe der Eindampfung erhöht sich der Gewichtsanteil Milchsäure von 0,15 auf 0,4. Nach der zweiten Stufe beträgt er 78 Prozent. In der dreibödigen Rektifikationskolonne wird der Milchsäuregehalt schließlich auf 95% vergrößert.

Die aufkonzentrierte Milchsäure wird in einer zweistufigen Polykondensation mit Hilfe von SnCl₂ als Katalysator zu einem Präpolymer mit mittlerem Molekulargewicht von 3400 g/mol polymerisiert. Im ersten Reaktor herrscht dabei Atmosphärendruck und eine Temperatur von 180'C. Der Druck im zweiten Reaktor beträt 50 mbar und die Temperatur 190°C.

Unter Beibehaltung des Vakuums und Erhöhung der Temperatur auf 225°C depolymerisiert anschließend das Präpolymer in einem Fallstromverdampfer zum Dilactid, das zyklische Dimer der Milchsäure. Der dampfförmige Produktstrom enthält 98% Dilactid, 1% Milchsäure und Lactoylmilchsäre und 1% Wasser. Durch Teilkondensation wird das Restwasser und ein Teil der Milchsäure vom Dilactid getrennt. Das Kondensat weist eine Konzentration an
OH-Gruppen von 0,04 mol/kg auf. In der anschließenden Rektifikation trennt man noch die restliche Lactoylmllchsäure und Milchsäure vom Dilactid, um die gewünschte Reinheit von 0,02 mol/kg zu erhalten. Diese Reinheit ist für ein Molekulargewicht des Polymers von 50 000 g/mol erforderlich. In den beiden folgenden Reaktoren findet die Ringöffnungspolymerisation statt. Unter Zusatz von 5 mol Zinnoctoat pro 100000 mol Dilactid und einer Temperatur von 195°C polymerisiert das Dilactid im ersten Reaktor zu einem Polylactid mit einem mittleren Molekulargewicht von 35000 g/mol, wobei 70% des Dilactids umgesetzt werden. Im zweiten Reaktor erhöht sich die Temperatur auf 215°C und das Molekulargewicht auf 50 000 g/mol. Der Umsatz an Dilactid steigt auf 90%. Die beiden Reaktoren stehen unter Atmosphärendruck.

In diesem Zustand enthält das Polylactid noch 10% Dilactid. Um ein stabiles Polylactid zu erhalten, muss der Dilactidanteil auf unter ein Prozent gesenkt werden. Dies geschieht ebenfalls zweistufig. In der ersten Stufe tritt das Polylactid in ein unter Vakuum (10 mbar) stehendes Fallrohr ein. Dabei verdampft ein Großteil des Dilactids, und die austretenden Schmelze weist noch 2% Monomer auf. Die Entmonomerisierung auf unter ein Prozent Dilactid geschieht in einem Scheibenreaktor, der unter einem Druck von 2,5 mbar steht. Die zur Verdampfung des restlichen Monomers erforderliche große Oberfläche wird durch sich drehende Scheiben erzeugt, die sich durch die Schmelze drehen und dabei Dilactid aus dem Schmelzeinneren an die Oberfläche befördern.

Das so erzeugte Polylactid hat ein Molekulargewicht von 50 000 g/mol, einen Monomergehalt von weniger als 1 Prozent und einen Schmelzpunkt von ca. 170°C. Das leicht gelbliche Polylactid besteht zu 95% aus L-Dilactid und zu 5% aus D-Dilactid.

**Tab.1: Vergleich zwischen den mit Lactobacillus paracasei unter kontinuierlichen Fermentationsbedingungen erreichten Milchsäurebitdungsproduktivitäten auf einem Glucose-MRS-Medium und einem MRS-Medium, bei dem Pepton durch alkalischen Roggenproteinextrakt ersetzt wurde.**

| | Durchflußrate (h⁻¹) | | |
|---|---|---|---|
| | 0,1 | 0,25 | 0,5 |
| Medium mit Hefeextrakt und Pepton | 3,4 g/lh | 13,2 g/lh | 17,0 g/lh |
| | | | |
| Medium mit Proteinextrakt und Hefeextrakt | 3,4 g/lh | 13,0 g/lh | 16,2 g/lh |

## Patentansprüche

1. Verfahren zur Herstellung von Polymilchsäure mit folgenden Schritten:
a) fermentative Gewinnung von Milchsäure aus stärkehaltigen landwirtschaftlichen Produkten, wobei diese einer Stärkehydrolyse unterzogen werden und der bei der Stärkehydrolyse anfallende Restfeststoff zusätzlich einer alkalischen Extraktion unterzogen und der erhaltene Proteinextrakt als Wuchsstoffquelle dem Fermentor zugeführt wird,
b) Hochreinigung der Milchsäure durch Ultrafiltration, Nanofiltration und/oder Elektrodialyse,
c) Aufkonzentrierung der Milchsäure und Herstellung eines Präpolymers,
d) zyklisierende Depolymerisation zum Dilactid,
e) Reinigung des Dilactids,
f) Ringoffnungspolymerisation des Dilactids,
g) Entmonomerisierung des Polylactids.

2. Verfahren nach Anspruch 1, **dadurch gekennzeichnet, dass** beim Verfahrensschritt a) ein Teil der ursprünglich mit der Nährlösung zugeführten und von den Bakterien genutzten externen Wuchsetoffkomponenten durch Lyse, die thermisch, durch Strahlen oder Enzyme realisiert wird, von gebildeter Überschußbiomasse direkt im Fermentationskreislauf den aktivierten Zellen wieder verfügbar gemacht wird.

3. Verfahren nach Anspruch 2, **dadurch gekennzeichnet, dass** das stärkehaltige landwirtschaftliche Produkt, vorzugsweise Getreide, gemahlen und ein erster Teil des Produktes einer Stärkehydrolyse unterzogen und die gewonnene Glucoselösung unter anaeroben Bedingungen in einem Fermentor mit einem Bakterium umgesetzt wird, wobei der zweite Teil des Produktes einer alkalischen Extraktion unterworfen und der erhaltene Proteinextrakt als Wuchsstoffquelle dem Fermentor zugeführt wird und der ungelöste stärkehaltige Rückstand der Stärkehydrolyse zugeführt wird.

4. Verfahren nach mindestens einem der Ansprüche 2 bis 3, **dadurch gekennzeichnet, dass** die bei der Fermentation erhaltene Überschußbiomasse in einem separaten Kreislauf geführt, dort lysiert und danach in den Fermentor zurückgeführt wird.

5. Verfahren nach einem der Ansprüche 2 bis 4, **dadurch gekennzeichnet, dass** die zur Konstanthaltung der Konzentration der Biomasse im Fermentor auf gewünschtem Niveau über einen Regelvorgang soviel Biomasse pro Zeiteinheit lysiert wird, wie Biomasse zugewachsen ist.

6. Verfahren nach mindestens einem der Ansprüche 2 bis 5, **dadurch gekennzeichnet, dass** die Stärkehydrolyse, die in Form eines zweistufigen enzymatischen Prozesses durchgeführt wird, mit der Proteinextraktgewinnung gekoppelt wird.

7. Verfahren nach Anspruch 6, **dadurch gekennzeichnet, dass** die Stärke in der ersten Stufe mit dem Enzym α-Amylase verflüssigt und in der zweiten Stufe mit dem Enzym Glucoamylase verzuckert wird.

8. Verfahren nach mindestens einem der Ansprüche 1 bis 7, **dadurch gekennzeichnet, dass** als landwirtschaftliches Getreide wie Roggen, Weizen, Gerste und/oder Triticalemehl sowie Mais, Reis und/oder Kassava eingesetzt wird.

9. Verfahren nach mindestens einem der Ansprüche 2 bis 8, **dadurch gekennzeichnet, dass** für die Fermentation Einzel- oder Mischkulturen der Stämme Lactobacillus, Lactococcus, Streptococcus, Enterococcus oder Pediococcus verwendet wird.

10. Verfahren nach mindestens einem der Ansprüche 2 bis 9, **dadurch gekennzeichnet, dass** das Hydrolysat und/oder der Proteinextrakt bzw. der Nährstoffextrakt sterilisiert werden.

11. Verfahren nach Anspruch 1, **dadurch gekennzeichnet, dass** das Hydrolysat und die Nährstoff- bzw. Proteinstoffextrakte getrennt sterilisiert werden.

12. Verfahren nach mindestens einem der Ansprüche 1 bis 10, **dadurch gekennzeichnet, dass** die Fermentation kontinuierlich durchgeführt wird und die Abtrennung und Reinigung der Milchsäure mit Membrantrennprozessen erfolgt.

13. Verfahren nach mindestens einem der Ansprüche 1 bis 12, **dadurch gekennzeichnet, dass** die Aufkonzentrierung (Verfahrensschritt c)) so durchgeführt wird, dass eine mindestens 90 %ige Milchsäure vorliegt.

14. Verfahren nach Anspruch 13, **dadurch gekennzeichnet, dass** die Aufkonzentrierung durch eine zweistufige Eindampfung und eine Hochkonzentration erfolgt, wobei die Kondensationswärme der zweiten Stufe zur Verdampfung in der 1. Stufe genutzt wird.

15. Verfahren nach mindestens einem der Ansprüche 1 bis 14, **dadurch gekennzeichnet, dass** die zyklisierende Depolymerisation (Verfahrensschritt d)) in einem Fallfilmverdampfer durchgeführt wird.

16. Verfahren nach mindestens einem der Ansprüche 1 bis 15, **dadurch gekennzeichnet, dass** beim Verfahrensschritt e) die Reinigung bis zu einer Hydroxylgruppenkonzentration < 25 meq durchgeführt wird.

17. Verfahren nach Anspruch 15, **dadurch gekennzeichnet, dass** die Lactidreinigung in einer Rektifikationskolonne erfolgt.

18. Verfahren nach mindestens einem der Ansprüche 1 bis 17, **dadurch gekennzeichnet, dass** die Ringöffnungpolymerisation des Dilactids (Verfahrensschritt f)) mit einer Katalysatorkonzentration von 2 x 10⁻⁴ bis 2 x 10⁻⁵ Mol pro Mol durchgeführt wird.

19. Verfahren nach einem der Ansprüche 1 bis 18, **dadurch gekennzeichnet, dass** vor der Entmonomerisierung ein Stabilisator zugegeben wird, der den Katalysator blockiert.

20. Vorrichtung zur Durchführung des Verfahrens nach mindestens einem der Ansprüche 1 bis 19, umfassend jeweils mindestens eine Mischeinrichtung (1), eine Hydrolysseinrichtung (2), die mit mindestens einem Proteinextraktor (11) verbunden ist, einen Fermentor (3), eine Hochreinigungseinrichtung (4), einen Aufkonzentrierer (5), eine Polykondensationseinrichtung (6), eine Depolymerisationsvörrichtung (7), eine Hochreinigungseinrichtung (8) für das Dilactid, einen Reaktor (9) für die Polymerisation und eine Entmonomerisierungsvorrichtung (10).

21. Vorrichtung nach Anspruch 20, **dadurch gekennzeichnet, dass** die Hydrolyseeinrichtung (2) ein im Außenkreislauf befindliches Ultrafiltrationsmodul (12) aufweist.

22. Vorrichtung nach mindestens einem der Ansprüche 20 oder 21, **dadurch gekennzeichnet, dass** dem mindestens einen Fermentor (3) mindestens eine Sterilisationsvorrichtung (13) vorgeschaltet ist.

23. Vorrichtung nach mindestens einem der Ansprüche 20 bis 22, **dadurch gekennzeichnet, dass** als Hochreinigungseinrichtung (4) nacheinandergeschaltet eine Nanofiltrationseinrichtung (15), eine monopolare Elektrodialyseeinrichtung (16) und eine bipolare Elektrodialyseeinrichtung (17) vorgesehen sind.

24. Vorrichtung nach mindestens einem der Ansprüche 20 bis 23, **dadurch gekennzeichnet, dass** die Aufkonzentrierungseiririchtung (5) dreistufig ausgebildet ist und aus nacheinander angeordneten Verdampfern (18) und (19) und einem nachgeschalteten Hochkonzentrator (20) besteht.

25. Vorrichtung nach mindestens einem der Ansprüche 20 bis 24, **dadurch gekennzeichnet, dass** die Vorkondensation (5) in zwei Reaktoren (21) und (22) mit außenliegendem Umlaufverdampfer erfolgt.

26. Vorrichtung nach mindestens einem der Ansprüche 20 bis 25, **dadurch gekennzeichnet, dass** die Depolymerisationsvorrichtung (7) ein Fallfilmverdampfer ist.

27. Vorrichtung nach mindestens einem der Ansprüche 20 bis 26, **dadurch gekennzeichnet, dass** die Hochreinigungseinrichtung (8) für die Dilactidreinigung mindestens eine Rektifikationskolonne ist.

28. Vorrichtung nach mindestens einem der Ansprüche 20 bis 27, **dadurch gekennzeichnet, dass** der Reaktor (9) für die Ringöffnungspolymerisation eine Rührkesselkaskade mit mindestens zwei Reaktoren ist.

29. Vorrichtung nach mindestens einem der Ansprüche 20 bis 28, **dadurch gekennzeichnet, dass** die Entmonomerisierungseinrichtung (10) aus einem Vakuumreaktor und einem Ringscheibenreaktor besteht.

30. Vorrichtung nach einem der Ansprüche 20 bis 29, **dadurch gekennzeichnet, dass** die Entmonomerisierungseinrichtung einen Dünnschichtverdampfer aufweist.

## Claims

1. Method for producing polylactic acid, with the following steps:
a) fermentative production of lactic acid from starch containing agricultural products, whereby the same are subjected to a starch hydrolysis, and whereby the residual solids generated during the starch hydrolysis are further subjected to an alkaline extraction and the resulting protein extract added to the fermenter as a growth agent source,
b) high cleaning of the lactic acid by means of ultrafiltration, nanofiltration and/or electrodialysis,
c) concentrating the lactic acid and producing a prepolymer,
d) cylising depolymerisation into a dilactide,
e) cleaning of the dilactide,
f) ring opening polymerisation of the dilactide,
g) demonomerisation of the polylactide.

2. Method according to Claim 1, **characterised in that** a part of the external growth components originally added with the nutrient solution and utilised by the bacteria are made available by means of lysis realised thermally with rays or enzymes from the formed excess biomass directly in the fermentation cycle of the active cells during method step a).

3. Method according to Claim 2, **characterised in that** the starch containing agricultural product, preferably grain, is ground and a first part of the product is subjected to a starch hydrolysis, and the glucose solution produced in this way transformed under anaerobic conditions in a fermenter with a bacterium, whereby the second part of the product is subjected to an alkaline extraction and the resulting protein extract added to the fermenter as a growth agent source, and the undissolved starch containing residue added to the starch hydrolysis.

4. Method according to at least one of the Claims 2 to 3, **characterised in that** the excess biomass generated during the fermentation is added to a separate cycle, lysised there, and then returned to the fermenter.

5. Method according to one of the Claims 2 to 4, **characterised in that** as much biomass as has been accumulated by the increase of the same is lysised per time unit by means of a control process for the desired level to be maintained for a constant concentration of the biomass in the fermenter.

6. Method according to at least one of the Claims 2 to 5, **characterised in that** the starch hydrolysis carried out in the form of a two-step enzymatic process is coupled with the protein extract generation.

7. Method according to Claim 6, **characterised in that** the starch is liquidised together with the enzyme α-amylasis during the first step, and sugared with the enzyme glucoamylase during the second step.

8. Method according to at least one of the Claims 1 to 7, **characterised in that** rye, wheat, barley and/or triticale flour as well as maize, rice and or cassava are used as agricultural grains.

9. Method according to at least one of the Claims 2 to 8, **characterised in that** individual or mixed cultures of the bases lactobacillus, lactococcus, streptococcus, enterococcus, or pediococcus are used for the fermentation.

10. Method according to at least one of the Claims 2 to 9, **characterised in that** the hydrolysate and/or the protein extract, e.g. the nutrient extract is sterilised.

11. Method according to Claim 1, **characterised in that** the hydrolysate and the nutrient, e.g. protein extracts are sterilised separately.

12. Method according to at least one of the Claims 1 to 10, **characterised in that** the fermentation is carried out continuously, and the separation and cleaning of the lactic acid is carried out with the aid of a membrane separation process.

13. Method according to at least one of the Claims 1 to 12, **characterised in that** the concentrating (method step c)) is carried out in such a way that an at least 90% lactic acid is produced.

14. Method according to Claim 13, **characterised in that** the concentrating is carried out by means of two-step steaming and high concentration, whereby the condensation heat of the second step is used for steaming during the 1. step.

15. Method according to at least one of the Claims 1 to 14, **characterised in that** the cyclising depolymerisation (method step d)) is carried out in a fall film evaporator.

16. Method according to at least one of the Claims 1 to 15, **characterised in that** the cleaning during method step e) is carried out up to a hydroxyl group concentration pf<25 meq.

17. Method according to Claim 15, **characterised in that** the lactide cleaning is carried out in a rectification column.

18. Method according to at least one of the Claims 1 to 17, **characterised in that** the ring opening polymerisation of the dilactide (method step f)) is carried out with a catalyst concentration of 2 x 10⁻⁴ to 2 x 10⁻⁵ mol per mol.

19. Method according to one of the Claims 1 to 18, **characterised in that** a stabiliser which blocks the catalyst is added prior to the demonomerisation.

20. Device for carrying out the method according to at least one of the Claims 1 to 19, each comprising at least one mixing means (1), a hydrolysis means (2) connected with at least one protein extractor (11), a fermenter (3), a high cleaning means (4), a concentrator (5), a polycondensation means (6), a depolymerisation means (7), a high cleaning means (8) for the dilactide, a reactor (9) for the polymerisation, and a demonomerisation means (10).

21. Device according to Claim 20, **characterised in that** the hydrolysis means (2) comprises an ultrafiltration module (12) in the outer cycle.

22. Device according to at least one of the Claims 20 or 21, **characterised in that** the at least one fermenter (3) is preceded by at least one sterilisation means (13).

23. Device according to at least one of the Claims 20 to 22, **characterised in that** a nanofiltration means (15), a monopolar electrodialysis means (16), and a bipolar electrodialysis means (17) are envisaged switched in series as a high cleaning means (4).

24. Device according to at least one of the Claims 20 to 23, **characterised in that** the concentrating means (5) is of a three-step design, and consists of evaporators (18) and (19) switched in series, and a subsequent high concentrator (20).

25. Device according to at least one of the Claims 20 to 24, **characterised in that** the pre-condensation (5) is carried out in two reactors (21) and (22) with an outer circulation evaporator.

26. Device according to at least one of the Claims 20 to 25, **characterised in that** the depolymerisation means (7) is a fall film evaporator.

27. Device according to at least one of the Claims 20 to 26, **characterised in that** the high cleaning means (8) for the dilactide cleaning is at least one rectification column.

28. Device according to at least one of the Claims 20 to 27, **characterised in that** the reactor (9) for the ring opening polymerisation is a stirring vessel cascade with at least two reactors.

29. Device according to at least one of the Claims 20 to 28, **characterised in that** the demonomerisation means (10) consists of a vacuum reactor and a ring disc reactor.

30. Device according to one of the Claims 20 to 29, **characterised in that** the demonomerisation means comprises a thin layer evaporator.

## Revendications

1. Procédé de fabrication d'acide polylactique comprenant les étapes suivantes :
a) obtention par fermentation d'acide lactique à partir de produits agricoles contenant de l'amidon, lesdits produits étant sont soumis à une hydrolyse de l'amidon, les matières solides restantes résultant de l'hydrolyse de l'amidon étant en outre soumises à une extraction alcaline et l'extrait protidique obtenu étant conduit à un fermenteur en tant que source de substances de croissance,
b) purification de l'acide lactique par ultrafiltration, nanofiltration et/ou électrodialyse,
c) concentration de l'acide lactique et réalisation d'un prépolymère,
d) dépolymérisation avec cyclisation pour obtenir un dilactide,
e) nettoyage du dilactide,
f) polymérisation avec ouverture annulaire du dilactide,
g) démonomérisation du polylactide.

2. Procédé selon la revendication 1, **caractérisé en ce que** lors de l'étape a) de la procédure, une partie des composants externes de substance de croissance, initialement amenés avec le bouillon de culture et utilisés par les bactéries, est de nouveau mise à disposition des cellules activées par l'excédent de biomasse qui s'est formé directement dans le cycle de fermentation grâce à une lyse réalisée thermiquement par des rayons ou des enzymes.

3. Procédé selon la revendication 2, **caractérisé en ce que** le produit agricole contenant de l'amidon, de préférence des céréales, est broyé, et une première partie du produit est soumise à une hydrolyse de l'amidon et la solution de glucose obtenue est déplacée dans des conditions anaérobies dans un fermenteur avec une bactérie, une seconde partie du produit est-elle soumise à une extraction alcaline et l'extrait protidique obtenu est conduit au fermenteur en tant que source de substances de croissance puis le reste non dissout contenant de l'amidon est conduit à l'hydrolyse de l'amidon.

4. Procédé selon l'une au moins des revendications 2 à 3, **caractérisé en ce que** l'excédent de biomasse obtenu lors de la fermentation est amené dans un cycle séparé, où il est soumis à une lyse puis ramené dans le fermenteur.

5. Procédé selon l'une des revendications 2 à 4, **caractérisé en ce que** pour maintenir à un niveau constant la concentration de biomasse dans le fermenteur au niveau souhaité par un processus de régulation, un volume de biomasse par unité de temps égal à celui accumulé est soumis à une lyse.

6. Procédé selon l'une au moins des revendications 2 à 5, **caractérisé en ce que** l'hydrolyse d'amidon, qui est réalisée sous la forme d'un processus enzymatique sur deux niveaux, est couplée à une obtention d'extrait protidique.

7. Procédé selon la revendication 6, **caractérisé en ce que** l'amidon est liquéfié dans le premier niveau avec l'enzyme α-amylase puis saccharifiée dans le second niveau avec l'enzyme glucoamylase.

8. Procédé selon l'une au moins des revendications 1 à 7, **caractérisé en ce que** des céréales agricoles telles que du seigle, du blé, de l'orge et/ou de la triticale ainsi que du maïs, du riz et/ou du manioc sont utilisées.

9. Procédé selon l'une au moins des revendications 2 à 8, **caractérisé en ce que** des cultures individuelles ou mélangées des souches de lactobacille, lactocoque, streptocoque, entérocoque ou pédiocoque sont utilisées pour la fermentation.

10. Procédé selon l'une au moins des revendications 2 à 9, **caractérisé en ce que** l'hydrolysat et/ou l'extrait protidique ou bien l'extrait de nutriment sont stérilisés.

11. Procédé selon la revendication 1, **caractérisé en ce que** l'hydrolysat et les extraits de nutriment et/ou de protéine sont stérilisés séparément.

12. Procédé selon l'une au moins des revendications 1 à 10, **caractérisé en ce que** la fermentation est réalisée de manière continue et la séparation et le lavage de l'acide lactique sont effectués avec des processus de séparation de membrane.

13. Procédé selon l'une au moins des revendications 1 à 12, **caractérisé en ce que** la concentration (étape c) du procédé) est réalisée de manière à avoir un acide lactique avec une concentration d'au moins 90%.

14. Procédé selon la revendication 13, **caractérisé en ce que** la concentration est obtenue par une évaporation sur deux niveaux et une forte concentration a lieu, moyennant quoi la chaleur de condensation du second niveau est utilisée pour l'évaporation dans le premier niveau.

15. Procédé selon l'une au moins des revendications 1 à 14, **caractérisé en ce que** la dépolymérisation avec cyclisation (étape d) du procédé) est réalisée dans un évaporateur à film descendant.

16. Procédé selon au moins l'une des revendications 1 à 15, **caractérisé en ce que** lors de l'étape e) du procédé, le nettoyage est réalisé jusqu'à l'obtention d'une concentration du groupe hydroxyle <25 meq.

17. Procédé selon la revendication 15, **caractérisé en ce que** le lavage des lactides a lieu dans une colonne de rectification.

18. Procédé selon l'une au moins des revendications 1 à 17, **caractérisé en ce que** la polymérisation à ouverture annulaire du dilactide (étape f) du procédé) est conduite avec une concentration du catalyseur de 2x10⁻⁴ jusqu'à 2x10⁻⁵ mol par mol.

19. Procédé selon l'une des revendications 1 à 18, **caractérisé en ce qu'**un stabilisateur est ajouté avant la démonomérisation, qui bloque le catalyseur.

20. Dispositif permettant de réaliser le procédé selon au moins l'une des revendications 1 à 19, comprenant au moins un dispositif de mélange (1), un dispositif d'hydrolyse (2), qui est relié à au moins un extracteur de protéine (11), un fermenteur (3), un dispositif de purification (4), un concentrateur (5), un dispositif de polycondensation (6), un dispositif de dépolymérysation (7), un dispositif de purification (8) pour le dilactide, un réacteur (9) pour la polymérisation et un dispositif de démonomérisation (10).

21. Dispositif selon la revendication 20, **caractérisé en ce que** le dispositif d'hydrolyse (2) présente un module d'ultrafiltration (12) se situant sur la boucle externe.

22. Dispositif selon l'une au moins des revendications 20 ou 21, **caractérisé en ce qu'**au moins un dispositif de stérilisation (13) est placé en amont du au moins un fermenteur (3).

23. Dispositif selon l'une au moins des revendications 20 à 22, **caractérisé en ce qu'**en tant que dispositif de purification, un dispositif de nanofiltration (15), un dispositif d'électrodialyse monopolaire (16), et un dispsoitf d'électrodialyse bipolaire (17) sont prévus agencés les uns derrière les autres.

24. Dispositif selon l'une au moins des revendications 20 à 23, **caractérisé en ce que** la concentration (5) est conçue de manière à avoir lieu sur trois niveaux et est constituée d'évaporateurs (18) et (19) et d'un concentrateur de très haute puissance (20) connecté en aval, tous agencés les uns derrière les autres.

25. Dispositif selon l'une au moins des revendications 20 à 24, **caractérisé en ce que** la précondensation (5) a lieu dans deux réacteurs (21) et (22) avec un évaporateur rotatif situé à l'extérieur.

26. Dispositif selon l'une au moins des revendications 20 à 25, **caractérisé en ce que** le dispositif de dépolymérisation (7) est un évaporateur à film tombant.

27. Dispositif selon l'une au moins des revendications 20 à 26, **caractérisé en ce que** le dispositif de purification (8) pour le lavage du dilactide est au moins une colonne de rectification.

28. Dispositif selon l'une au moins des revendications 20 à 27, **caractérisé en ce que** le réacteur (9) pour la polymérisation à ouverture annulaire est une cascade de cuves malaxeuses dotée d'au moins deux réacteurs.

29. Dispositif selon l'une au moins des revendications 20 à 28, **caractérisé en ce que** le dispositif de démonomérisation (10) est constitué d'un réacteur à vide et d'un réacteur à meules annulaires.

30. Dispositif selon l'une des revendications 20 à 29, **caractérisé en ce que** le dispositif de démonomérisation présente un évaporateur à couche mince.
